# EUROPEAN PATENT APPLICATION

(11) **EP 4 821 461 A1**
(43) Date of publication of application: **14.10.2026**
(21) Application number: 24928195.7
(22) Date of filing: 10.12.2024
(51) Int. Cl.: A61C 7/10

(54) **EXPANSION ORTHOSIS WORKING END AND EXPANSION ORTHOSIS**

(30) Priority: 06.03.2024 CN 202420435056 U
(71) Applicant: Dragon Crown Medical Co., Ltd., Jinan, Shandong 250101 (CN)
(72) Inventor: LI, Weiming, Jinan, Shandong 250101 (CN); PAN, Huihui, Jinan, Shandong 250101 (CN)
(74) Representative: Sun, Yiming
(86) International application number: PCT/CN2024/137992
(87) International publication number: WO 2025/185266

(57) **Abstract**

A distal assembly of orthopedic expander and orthopedic expander provided by the present invention relate to technical field of medical devices. The distal assembly of an orthopedic expander comprises an outer tube (1) and a pull rod (2), a front end of the pull rod (2) is provided with an elastic piece (3), the outer tube (1) is provided with a clearance opening (11); a rear end of the elastic piece (3) is provided with a first bending portion (31). A mounting groove (23) divides a front end portion of the pull rod (2) into a first clamping plate (21) and a second clamping plate (22), and the mounting groove (23) sequentially comprises a clamping portion (231) and a limiting portion (232). The pull rod (2) is sleeved with a locking ring (4), and the first clamping plate (21) and the second clamping plate (22) clamp the elastic piece (3).

## Description

### TECHNICAL FIELD

The present invention relates to the technical field of medical devices, and specifically relates to a distal assembly of an orthopedic expander and an orthopedic expander.

### BACKGROUND

A shape memory alloy is a material having a shape memory effect through thermoelasticity and martensitic transformation and reverse transformation thereof, so far, more than 50 kinds of alloys having a shape memory effect have been found, and are widely applied in multiple fields such as aerospace, mechatronics, biomedicine, bridge construction, and automotive industry. A nickel-titanium alloy, as a kind of the shape memory alloy, in addition to having a shape memory effect and superelasticity, also has characteristics of good biocompatibility, and is able to meet biological requirements, therefore is widely applied in the medical field.

The nickel-titanium alloy is a hard material in a cold working state, and a titanium oxide layer on a surface causes the nickel-titanium alloy to have extremely strong abrasiveness, therefore, difficulty in machining is high, and the nickel-titanium alloy cannot be machined by traditional machining technologies such as milling, turning, or drilling.

However, currently mounting structures of elastic pieces in a distal assembly of an orthopedic expander all require machining the elastic pieces, therefore, a material with poor machining performance (for example, nickel-titanium alloy) cannot be applied to the orthopedic expander as the elastic piece. Based on this, the elastic piece of the current orthopedic expander still adopts a stainless steel material with better machining performance.

### SUMMARY

In view of the above problems, the present invention provides a distal assembly of an orthopedic expander, the distal assembly solves a technical problem that a material with poor machining performance cannot be applied to an orthopedic expander as an elastic piece by improving the elastic piece and a mounting structure of the elastic piece. At the same time, the present invention also provides an orthopedic expander having the distal assembly of the orthopedic expander, and achieves the same technical effects, which will not be repeated herein.

Technical solutions adopted by the present invention to solve the technical problems of the present invention are as follows:
A distal assembly of an orthopedic expander, comprising an outer tube and a pull rod disposed in the outer tube, a front end of the pull rod is provided with an elastic piece, the outer tube is provided with a clearance opening; when pushing the pull rod forward, the elastic piece can bulge from the clearance opening; a rear end of the elastic piece is provided with a first bending portion;
a mounting groove disposed on the pull rod divides a front end portion of the pull rod into a first clamping plate and a second clamping plate; the mounting groove sequentially comprises a clamping portion and a limiting portion for accommodating the first bending portion from front to back;
the pull rod is sleeved with a locking ring, and the first clamping plate and the second clamping plate clamp the elastic piece under a locking action of the locking ring.

Further, in a free state, a width of the clamping portion of the mounting groove gradually increases from back to front.

Further, the front end of the pull rod is provided with two elastic pieces, and the first bending portions of the two elastic pieces are in a back-to-back state.

Further, a partition plate is disposed between the two elastic pieces in the outer tube.

Further, an end of the elastic piece facing away from the pull rod is provided with a second guiding slope; when suspended ends of the two elastic pieces abut against each other, the two second guiding slopes collectively form a sharp-angled depression.

Further, two sides of a suspended end of the partition plate are respectively provided with a first guiding slope, and the two first guiding slopes collectively form a sharp-angled structure.

Further, the elastic piece is provided with a pre-bent portion.

Further, a front end of the elastic piece is provided with a second bending portion; when the elastic piece is mounted to the pull rod, suspended ends of the two second bending portions abut against each other top to top, the second bending portion extends backward and arches up, thereby forming the pre-bent portion.

An orthopedic expander, comprising the distal assembly according to any one of claims 1 to 8.

Beneficial effects of the present invention are as follows:
A distal assembly of an orthopedic expander provided by an embodiment of the present invention is improved on an elastic piece and a mounting structure of the elastic piece. The improved elastic piece and the mounting structure of the improved elastic piece realized mounting and fixing of the elastic piece without needing to perform machining such as milling, turning, or drilling on the elastic piece, which solved the technical problem that a material with poor machining performance cannot be applied to an orthopedic expander as an elastic piece, and provided a prerequisite for application of a nickel-titanium alloy to the orthopedic expander. And since a nickel-titanium alloy has a shape memory effect, superelasticity, and good biocompatibility, applying the nickel-titanium alloy to the orthopedic expander as the elastic piece can improve the performance and service life of the orthopedic expander.

### BRIEF DESCRIPTION OF THE DRAWINGS

FIG. 1 is a cross-sectional view of a distal assembly of an orthopedic expander provided by an embodiment of the present invention;
FIG. 2 is an enlarged structural schematic diagram of part A in FIG. 1;
FIG. 3 is an enlarged structural schematic diagram of part B in FIG. 1;
FIG. 4 is an enlarged structural schematic diagram of part C in FIG. 1;
FIG. 5 is a mounting structural schematic diagram of an elastic piece;
FIG. 6 is an enlarged structural schematic diagram of part D in FIG. 5;
FIG. 7 is a structural schematic diagram of a front end portion of a pull rod;
FIG. 8 is a structural schematic diagram of an elastic piece.

In the drawings: 1, outer tube; 11, clearance opening;
2, pull rod; 21, first clamping plate; 22, second clamping plate; 23, mounting groove; 231, clamping portion; 232, limiting portion;
3, elastic piece; 31, first bending portion; 32, second bending portion; 33, second guiding slope; 34, pre-bent portion;
4, locking ring;
51, partition plate; 511, first guiding slope; 52, connecting block.

### DETAILED DESCRIPTION OF THE DISCLOSURE

In order to enable personnel in this technical field to better understand the technical solutions in the present invention, the technical solutions in embodiments of the present invention will be described in detail below in combination with the drawings in the embodiments of the present invention, and the described embodiments are merely a part of embodiments of the present invention rather than all the embodiments. All other embodiments obtained by those skilled in the art based on the embodiments provided by the present invention through means such as combination, splitting, and recombination do not exceed a protection scope of the present invention.

To facilitate description, a holding end of an orthopedic expander is now defined as a rear end, and an end opposite to the holding end is defined as a front end.

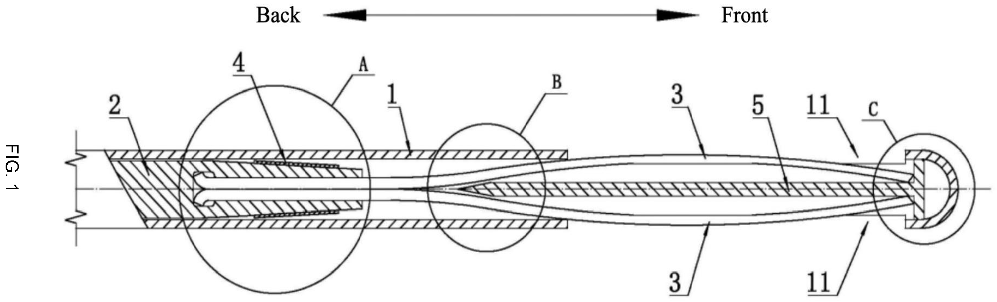

As shown in FIG. 1, a distal assembly of an orthopedic expander comprises an outer tube 1 and a pull rod 2 disposed in the outer tube 1, and the pull rod 2 can slide back and forth relative to the outer tube 1. A front end of the pull rod 2 is fixedly provided with an elastic piece 3, and a front end portion of the elastic piece 3 extends to a front side of the pull rod 2 to be in a suspended state. A side wall of the outer tube 1 is provided with a clearance opening 11 corresponding to the elastic piece 3. When a front end of the elastic piece 3 abuts against the outer tube 1, and the pull rod 2 is pushed forward, a middle portion of the elastic piece 3 can bulge from the clearance opening 11, at this time, rotating the orthopedic expander can scrape off bone spurs and the like in a surgical area through the bulging elastic piece 3.

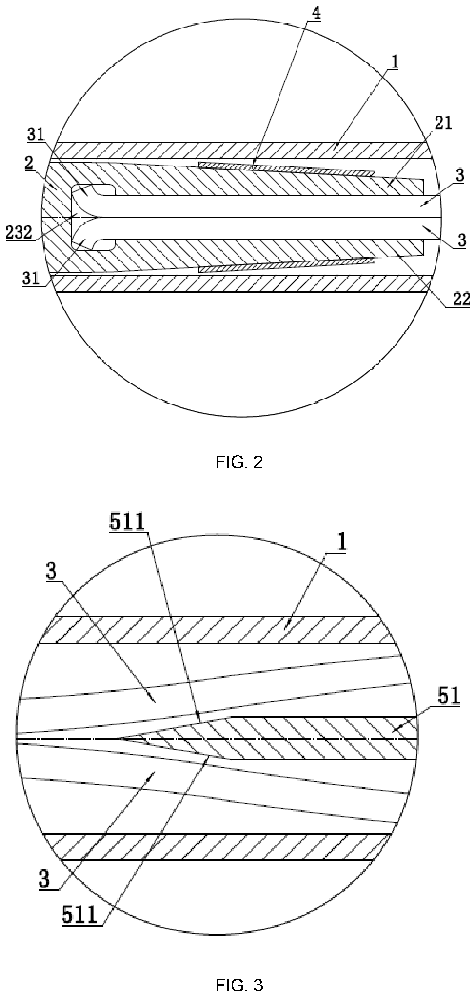

As shown in FIG. 2, FIG. 5, and FIG. 7, a rear end of the elastic piece 3 is provided with a first bending portion 31 bent to one side. The front end of the pull rod 2 is provided with a mounting groove 23, and the mounting groove 23 divides a front end portion of the pull rod 2 into a first clamping plate 21 and a second clamping plate 22. The mounting groove 23 sequentially comprises a clamping portion 231 and a limiting portion 232 for accommodating the first bending portion 31 from front to back. The front end of the pull rod 2 is sleeved with a locking ring 4, and the first clamping plate 21 and the second clamping plate 22 clamp the elastic piece 3 under a locking action of the locking ring 4, and the elastic piece 3 is clamped by the clamping portion 231 of the mounting groove 23.

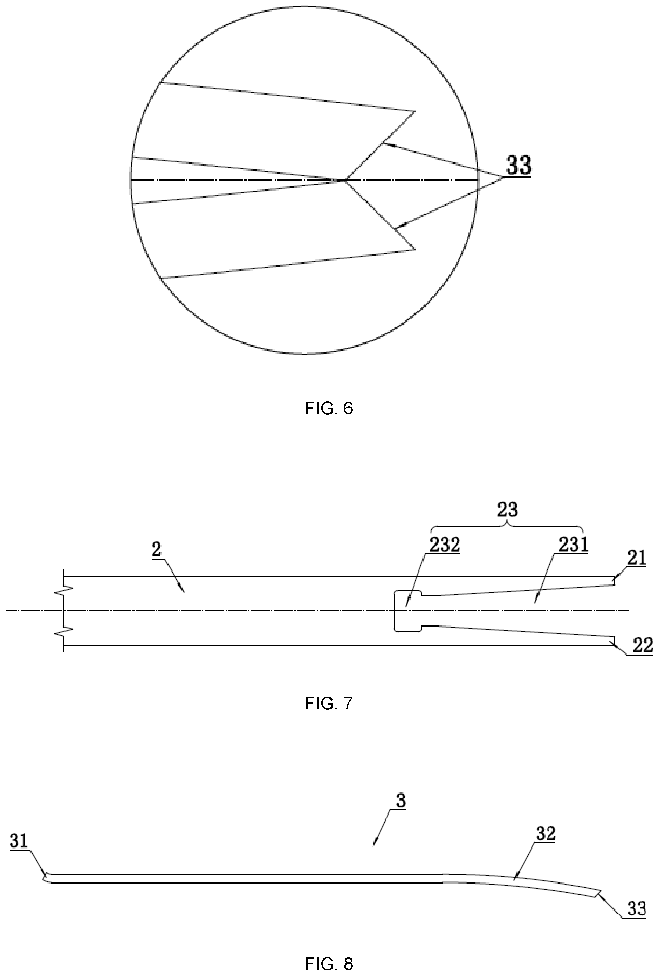

Further, in order to enable the first clamping plate 21 and the second clamping plate 22 to reliably clamp the elastic piece 3, as shown in FIG. 2 and FIG. 7, in a free state, a width of the clamping portion 231 of the mounting groove 23 gradually increases from back to front, and has a horn-shaped structure. When the locking ring 4 is sleeved on the pull rod 2, the first clamping plate 21 and the second clamping plate 22 will undergo elastic deformation, thereby clamping the elastic piece 3. In this way, a clamping force of the first clamping plate 21 and the second clamping plate 22 can be adjusted by pushing the locking ring 4 backward, so that the first clamping plate 21 and the second clamping plate 22 can reliably clamp the elastic piece 3.

The locking ring 4 can have a cylindrical structure or a conical structure. As a specific embodiment, the locking ring 4 in the present embodiment has a conical structure.

Here, the front end of the pull rod 2 can be provided with one elastic piece 3 or two elastic pieces 3. When two elastic pieces 3 are provided, as shown in FIG. 2, the first bending portions 31 of the two elastic pieces 3 are in a back-to-back state. Correspondingly, the side wall of the outer tube 1 is provided with two clearance openings 11, and the two clearance openings 11 correspond to the elastic pieces 3 one to one.

As a specific embodiment, the front end of the pull rod 2 in the present embodiment is provided with two elastic pieces 3.

Further, in order to ensure that the elastic piece 3 can bulge from a corresponding clearance opening 11, as shown in FIG. 1, a partition plate 51 is disposed between the two elastic pieces 3 in the outer tube 1, an end of the partition plate 51 facing away from the pull rod 2 is fixedly connected to a blind end of the outer tube 1, and an end of the partition plate 51 facing the pull rod 2 is in a suspended state.

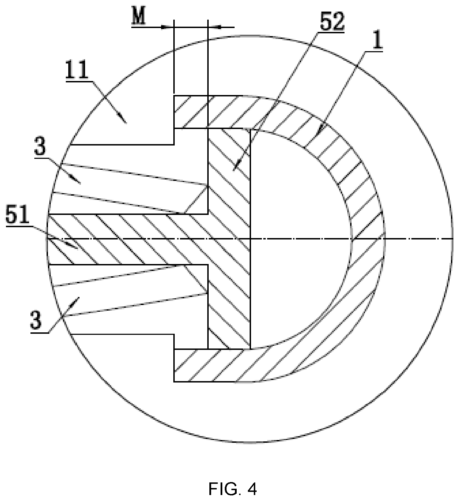

As a specific embodiment, as shown in FIG. 4, an end of the partition plate 51 facing away from the pull rod 2 in the present embodiment is provided with a connecting block 52, and a cross-sectional shape of the connecting block 52 matches a cross-sectional shape of an inner cavity of the outer tube 1, and the connecting block 52 is fixedly connected to the outer tube 1 by means of welding. Exemplarily, the connecting block 52 and the partition plate 51 form an integral structure.

Further, as shown in FIG. 4, a rear side surface of the connecting block 52 is located at a front side of a front side wall of the clearance opening 11, and a certain distance M exists between the rear side surface of the connecting block 52 and the front side wall of the clearance opening 11. In this way, a suspended end of the elastic piece 3 can be prevented from escaping from the clearance opening 11.

Further, as shown in FIG. 3 and FIG. 6, two sides of a suspended end of the partition plate 51 are respectively provided with a first guiding slope 511, the two first guiding slopes 511 are symmetrically arranged, and collectively form a sharp-angled structure. An end of the elastic piece 3 facing away from the pull rod 2 is provided with a second guiding slope 33, when suspended ends of the two elastic pieces 3 abut against each other, the two second guiding slopes 33 collectively form a sharp-angled depression.

A reason for such a design is that, when the pull rod 2 is pulled backward so that the elastic piece 3 is separated from the partition plate 51, pushing the pull rod 2 forward again enables the partition plate 51 to smoothly enter between the two elastic pieces 3, avoiding a problem of jamming due to abutting against each other top to top.

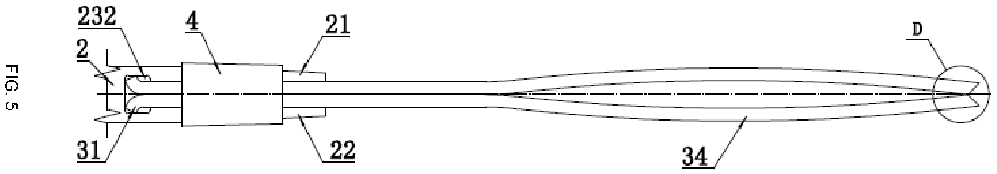

Further, as shown in FIG. 5, the elastic piece 3 is provided with a pre-bent portion 34, the pre-bent portion 34 faces a corresponding clearance opening 11, and when the pull rod 2 is pushed forward, the elastic piece 3 can continue to bend along a bending tendency of the pre-bent portion 34, and finally bulges from the clearance opening 11.

A reason for such a design is that, if the elastic piece 3 is a flat and straight structure, when the pull rod 2 is pushed against at the beginning to bend the elastic piece 3, a large force needs to be applied, which is not only inconvenient to operate, but may also similarly cause a situation of jamming due to abutting against each other top to top.

Further, as shown in FIG. 8, a front end of the elastic piece 3 is provided with a second bending portion 32 bent to one side, and a bending direction of the second bending portion 32 is opposite to a bending direction of the first bending portion 31. As shown in FIG. 5, when the elastic piece 3 is mounted to the pull rod 2, suspended ends of the two second bending portions 32 abut against each other top to top, the second bending portion 32 extends backward and arches up, thereby forming the pre-bent portion 34.

An orthopedic expander comprises an operation handle and a distal assembly disposed on the operation handle, and the distal assembly is the distal assembly according to any one of the above embodiments, which will not be repeated herein.

All other embodiments obtained by those skilled in the art based on the embodiments provided by the present invention through means such as combination, splitting, and recombination do not exceed a protection scope of the present invention.

The above specific embodiments describe purposes, technical solutions, and beneficial effects of the embodiments of the present invention in detail. The above are merely specific embodiments of the embodiments of the present invention, and are not intended to limit the protection scope of the embodiments of the present invention. That is, any modifications, equivalent replacements, improvements, and the like made based on the embodiments of the present invention shall be included in the protection scope of the embodiments of the present invention.

## Claims

1. A distal assembly of an orthopedic expander, comprising an outer tube (1) and a pull rod (2) disposed in the outer tube (1), wherein a front end of the pull rod (2) is provided with an elastic piece (3), the outer tube (1) is provided with a clearance opening (11); when the pull rod (2) is pushed forward, the elastic piece (3) can bulge from the clearance opening (11); **characterized in that** a rear end of the elastic piece (3) is provided with a first bending portion (31);
a mounting groove (23) disposed on the pull rod (2) divides a front end portion of the pull rod (2) into a first clamping plate (21) and a second clamping plate (22), the mounting groove (23) sequentially comprises a clamping portion (231) and a limiting portion (232) for accommodating the first bending portion (31) from front to back;
the pull rod (2) is sleeved with a locking ring (4), and the first clamping plate (21) and the second clamping plate (22) clamp the elastic piece (3) under a locking action of the locking ring (4).

2. The distal assembly of an orthopedic expander according to claim 1, **characterized in that** in a free state, a width of the clamping portion (231) of the mounting groove (23) gradually increases from back to front.

3. The distal assembly of an orthopedic expander according to claim 1, **characterized in that** the front end of the pull rod (2) is provided with two elastic pieces (3), and the first bending portions (31) of the two elastic pieces (3) are in a back-to-back state.

4. The distal assembly of an orthopedic expander according to claim 3, **characterized in that** a partition plate (51) is disposed between the two elastic pieces (3) in the outer tube (1).

5. The distal assembly of an orthopedic expander according to claim 4, **characterized in that** an end of the elastic piece (3) facing away from the pull rod (2) is provided with a second guiding slope (33); when suspended ends of the two elastic pieces (3) abut against each other, the two second guiding slopes (33) collectively form a sharp-angled depression.

6. The distal assembly of an orthopedic expander according to claim 4 or 5, **characterized in that** two sides of a suspended end of the partition plate (51) are respectively provided with a first guiding slope (511), and the two first guiding slopes (511) collectively form a sharp-angled structure.

7. The distal assembly of an orthopedic expander according to claim 3, **characterized in that** the elastic piece (3) is provided with a pre-bent portion (34).

8. The distal assembly of an orthopedic expander according to claim 7, **characterized in that** a front end of the elastic piece (3) is provided with a second bending portion (32); when the elastic piece (3) is mounted to the pull rod (2), suspended ends of the two second bending portions (32) abut against each other top to top, the second bending portion (32) extends backward and arches up, thereby forming the pre-bent portion (34).

9. An orthopedic expander, **characterized by** comprising the distal assembly according to any one of claims 1 to 8.
